(19) Europäisches Patentamt
European Patent Office
Office européen des brevets

(11) Veröffentlichungsnummer: **0 360 077 B1**

(12) # EUROPÄISCHE PATENTSCHRIFT

(45) Veröffentlichungstag der Patentschrift: **21.07.93**

(21) Anmeldenummer: **89116496.4**

(22) Anmeldetag: **07.09.89**

(51) Int. Cl.5: **A61K 31/00**, A61K 31/38, A61K 31/40, A61K 31/45, A61K 31/50, A61K 31/505, A61K 31/54

(54) **Verwendung von Serotonin-Antagonisten bei der Behandlung von Apoplexia cerebri.**

(30) Priorität: **20.09.88 DE 3831888**

(43) Veröffentlichungstag der Anmeldung:
**28.03.90 Patentblatt 90/13**

(45) Bekanntmachung des Hinweises auf die Patenterteilung:
**21.07.93 Patentblatt 93/29**

(84) Benannte Vertragsstaaten:
**AT BE CH DE ES FR GB GR IT LI LU NL SE**

(56) Entgegenhaltungen:

**JOURNAL OF NEUROLOGY, NEUROSURGERY, AND PSYCHIATRY, Band 44, Nr. 2, 1981, Seiten 140-143; M.J.G. HARRISON et al.: "Role of 5HT in the morbidity of cerebral infarction-a study in the gerbil troke model"**

(73) Patentinhaber: **Troponwerke GmbH & Co. KG**
**Berliner Strasse 156**
**W-5000 Köln 80(DE)**

(72) Erfinder: **Traber, Jörg, Dr.**
**Löwenburgstrasse 12**
**W-5204 Lohmar 21(DE)**
Erfinder: **Bielenberg, Gerhard-Wilhelm, Dr.**
**Albert-Schweitzer-Strasse 1**
**W-3572 Amöneburg 3(DE)**

(74) Vertreter: **Mann, Volker, Dr. et al**
**c/o Bayer AG Konzernverwaltung RP Patentabteilung**
**W-5090 Leverkusen 1, Bayerwerk (DE)**

EP 0 360 077 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**INTENSIVE CARE MEDICINE**, Band 9, 1983, Seiten 123-126, Springer-Verlag; H. VAN AKEN et al.: "The influence of urapidil, a new antihypertensive agent, on cerebral perfusion pressure in dogs with and without intracranial hypertension"

**BRAIN RESEARCH**, Band 435, Nr. 1-2, 1987, Seiten 305-309, Elsevier Science Publishers B.V. (Biomedical Division); J.A. ZIVIN et al.: "A model for quantitative evaluation of embolic stroke therapy"

**NEUROLOGY**, Band 35, Nr. 4, April 1985, Seiten 584-587; J.A. ZIVIN: "Cyproheptadine reduces or prevents ischemic central nervous system damage"

## Beschreibung

Die Erfindung betrifft die Verwendung von Wirkstoffen, mit Serotonin-agonistischer Wirkung, die bei der Bindung an $5HT_{1A}$-Rezeptoren eine Bindungsstärke von kleiner als 10 000 nmol/l aufweisen, zur Herstellung von Arzneimitteln zur Behandlung von Apoplexia cerebri.

Apoplexia cerebri (Stroke, Schlaganfall, Gehirnschlag) ist eine Folge einer plötzlichen Durchblutungsstörung eines menschlichen Gehirnbereichs mit nachfolgenden Funktionsausfällen, mit entsprechenden neurologischen und/oder psychischen Symptomen. Die Ursachen für Apoplexia cerebri können in Hirnblutungen (z.B. nach einem Gefäßriß bei Hypertonie, Arteriosklerose und apoplektischem Aneurysma) und Ischämien (z.B. durch eine Blutdruckabfallkrise oder Embolie) liegen. Die Funktionsausfälle im Gehirn führen zu einer Degeneration und/oder Abtötung der Gehirnzellen (Journal of Cerebral Blood Flow and Metabolism 1: 155 bis 185 (1981)).

Es wurde die Verwendung von Wirkstoffen mit Serotonin-agonistischer Wirkung, die bei der Bindung an $5HT_{1A}$-Rezeptoren eine Bindungsstärke von kleiner als 10 000 nmol/l aufweisen, zur Herstellung von Arzneimittel zur Behandlung von Apoplexia cerebri gefunden.

Die erfindungsgemäßen Wirkstoffe bewirken überraschenderweise sowohl bei einer prophylaktischen als auch bei einer nach der Apoplexia cerebri erfolgenden (therapeutischen) Behandlung eine starke Herabsetzung der Degeneration und/oder der Abtötung der Gehirnzellen und verhindern oder vermindern die Funktionsausfälle des Gehirns.

Wirkstoffe mit Serotonin-agonistischer Wirkung können durch Bestimmung der Hemmung der Forskolin-stimulierten Adenylat-Cyclaseaktivität ($EC_{50}$-Wert) durch den betreffenden Wirkstoff identifiziert werden (J. Pharmacol Exp. Ther. 238, 248-253 (1986)). $5HT_{1A}$-Liganden mit agonistischer bzw. partiell agonistischer Wirkung inhibieren die Forskolin-stimulierte Adenylat-Cyclase, Wirkstoffe, die die Enzymaktivität vermindern, haben eine Serotonin-agonistische bzw. partiell Serotonin-agonistische Wirkung. Der genannte Adenylat-Cyclase-Hemmungstest kann beispielsweise wie folgt durchgeführt werden:
Rattenhippocampus-Membranen werden unter geeigneten Bedingungen mit $\alpha$-$^{32}$P-ATP und Forskolin in Ab- und Anwesenheit von erfindungsgemäßen Verbindungen inkubiert. Nach Abstoppen der Reaktion wird das radioaktiv markierte cycloAMP isoliert und quantitativ bestimmt. Daraus wird die Enzymaktivität berechnet.

Die Bindungsstärke (Inhibitionskonstante bzw. $K_i$-Wert) ist ein Maß für die Wechselwirkungen zwischen einem Wirkstoff und den $5HT_{1A}$-Serotonin-Rezeptoren (Molecular Pharmacology 16, 687-699 (1979); J. Neurochem. 36, 220-226, 1981).

Die Bindungsstärke kann beispielsweise wie folgt bestimmt werden:
Kalbshippocampus-Membranen werden mit $^{3}$H-Serotonin in An- und Abwesenheit von erfindungsgemäßen Substanzen inkubiert. Die Reaktion wird durch Filtration gestoppt und die auf den Filtern verbleibende Radioaktivität gemessen. Aus den erhaltenen Verdrängungskurven werden $IC_{50}$-Werte bzw. Inhibitionskonstanten $K_i$ berechnet.

Bevorzugte Wirkstoffe mit Serotonin-agonistischer Wirkung, die bei der Bindung an $5HT_{1A}$-Rezeptoren eine Bindungsstärke von kleiner als 10 000 nmol/l aufweisen, werden z.B. beschrieben in SCRIP's Serotonin Report, PJB-Publications (1988) und bei J. Fozard, Trends in Pharmacological Sciences 8, 501 (1987).

Hierzu gehören beispielsweise Verbindungen aus den Substanzklassen der
Aryl- und Hetaryl-piperazine (bekannt auch u.a. aus DE-A 220 873; DE-A 3 321 969; EP-A 82 402; DE-A 3 529 872; DE-A 3 248 160),
Aminotetrahydrobenzindole (DE-A 3 346 573; EP-A 153 083; EP-A 162 695),
Indolamine (u.a. Fozard, Trends in Pharmacological Sciences 8, 501 (1987); Scrip's Serotonin-Report, PJB-Publications (1988); EP-A 236 930, DE-A 3 131 728; DE-A 2 940 687; DE-A 3 320 521),
Aminoalkyl-benzodioxane (u.a. Fozard, Trends in Pharmacological Sciences 8, 501 (1987); Scrip's Serotonin Report, PJB-Publications (1988); EP-A 236 930; EP-A 170 213),
Amino-tetraline (u.a. Fozard, Trends in Pharmacological Sciences 8, 501 (1987); Scrip's Serotonin-Report, PJB-Publications (1988); EP-A 41 488; EP-A 236 930),
Amino-chromane und -thiopyrane (z.B. EP-A 222 996),
Indolyl-alkyl-piperidine (DE-A 3 430 284),
Tetrahydropyridine (z.B. aus Fozard, Trends in Pharmacological Sciences 8, 501 (1987); Scrip's Serotonin-Report, PJB-Publications (1988); EP-A 3 199).

Aus der Gruppe der Aryl- und Hetaryl-piperazin sind insbesondere die 2-Pyrimidinyl-1-piperazin-Derivate der Formel I

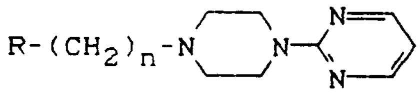

(I),

bzw, die Aryl-1-piperazin-derivate der Formel II

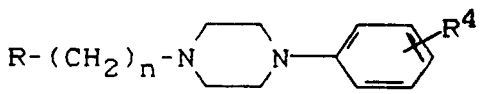

(II)

in der

n für eine der Zahlen 2, 3, 4, 5 oder 6 steht
R für einen der Reste

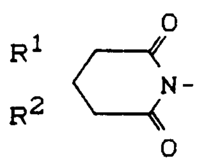

oder

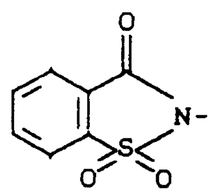

oder

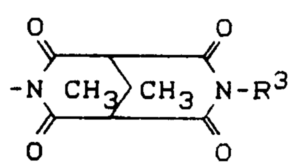

oder

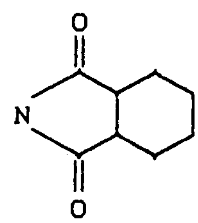

oder

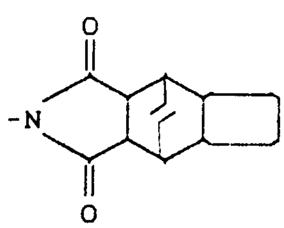

oder

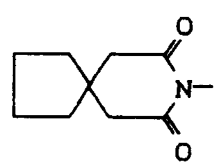

steht,

worin

$R^1$ und $R^2$ gleich oder verschieden sind und Wasserstoff oder Niederalkyl ($C_1$ bis etwa $C_6$) bedeutet,
$R^3$ für Alkyl ($C_1$ bis ca. $C_6$) oder Alkenyl steht und
$R^4$ für Wasserstoff, Alkoxy ($C_1$ bis $C_6$) oder Halogen steht,

und/oder deren Salze,
bevorzugt.

Außerdem seien noch (+)-N-[2-[4-[2,3-Dihydro-2-(hydroxymethyl)-1,4-benzodioxin-5-yl]-1-piperazinyl]-ethyl]-4-fluorobenzamidhydrochlorid (INN: Flesinoxanhydrochlorid) und
6-[[3-[4-[o-Methoxyphenyl]-1-piperazinyl]propyl]amino]-1,3-dimethyluracil [INN: Urapidil] genannt.

Beispielsweise seien die folgenden Wirkstoffe genannt: 8-[4-N-[4-(2-Pyrimidinyl)-1-piperazinyl]-butyl]-8-azaspiro[4,5]-decan-7,9-dionhydrochlorid (nach INN: Buspiron), 4,4-Dimethyl-1-[4-[4-(2-pyrimidinyl)-1-piperazinyl]-butyl]-2,6-piperidindionhydrochlorid (nach INN: Gepiron), 2-(4-(4-(2-Pyrimidinyl)-1-piperazinyl)-butyl)-1,2-benzoisothiazol-3(2H)-on-1,1-dioxidhydrochlorid (nach INN: Ipsapiron), 3a, 4, 4a, 6a, 7, 7a-hexahydro-2-[4-[4-(2-pyrimidinyl)-1-piperazinyl]-butyl]-4,7-etheno-1H-cyclobutano[f]isoindol-1,3(2H)-diondihydrochlorid-sesquihydrat (WY-47,846), N-[4-[4-(2-pyrimidinyl)-1-piperazinyl]butyl]-bicyclo[2.2.1]-heptan-2,3-di-exo-carboximid (SM 3997), 2-(4-(4-(2-Pyrimidinyl)-1-piperazinyl-propyl)-1,2-benzoisothiazol-3-(2H)-on-1,1-dioxidhydrochlorid, 3-Butyl-9,9-dimethyl-7-[4-[4-[2-methoxyphenyl)-1-piperazinyl]butyl]-3,7-diazabicyclo[3,2,1]nonan-2,4,6,8-tetraon (KC 9172).

Insbesondere bevorzugt wird Ipsapiron.

Die Piperazinderivate sind an sich bekannt (DE-A-3 321 969; Fozard, Trends in Pharmacological Sciences 8, 501 (1987); Scrip-Report, PJB-Publications (1988); EP-A 82 402; EP-A 220 873; DE-A 3 529 872; DE-A 3 248 160) und können beispielsweise durch Umsetzung eines entsprechenden Benzothiazols mit (Piperazinyl)-Pyrimidinen hergestellt werden.

Aus der Gruppe der Tetrahydrobenzindole sind insbesondere die 1,3,4,5-Tetrahydrobenz[c,d]indole der Formel

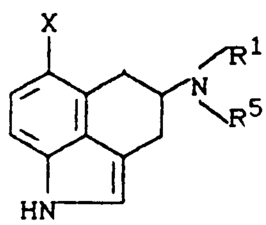

(II),

in welcher

X -für H, $OCH_3$, OH, $SCH_3$, Halogen, CN oder $CONH_2$ steht,

$R^1$ die oben angegebene Bedeutung hat, und

$R^5$ die oben für $R^1$ angegebene Bedeutung hat oder für -Y-Z steht, wobei

Y für eine geradkettige, gesättigte oder ungesättigte Alkylenkette mit bis zu 6 Kohlenstoffatomen steht, und

Z für eine Amino, Alkoxy ($C_1$ bis $C_6$), Sulfonamido, Carbonamido-Gruppe oder einen Heterocyclus

und/oder deren Salze

bevorzugt.

Beispielsweise seien die folgenden Wirkstoffe genannt:

4-(N,N-Dipropylamino)-6-methoxy-1,3,4,5-tetrahydrobenz-[c,d]indol, 4-[4-(N-1,2-benzisothiazol-3(2H)-on-1,1-dioxido)]butylamino-6-methoxy-1,3,4,5-tetrahydrobenz-[c,d]indol-hydrochlorid.

Als heterocyclische Reste sei z.B. 1,1-Dioxido-3-oxo-2H-1,2-benzisothiazol-2-yl oder 4,4-Dimethyl-2,6-dioxopiperidin-1-yl genannt.

Insbesondere bevorzugt wird hiervon 4-(N,N-dipropylamino)-6-methoxy-1,3,4,5-tetrahydrobenz[c,d]indol.

Die Tetrahydrobenzindol-Derivate sind an sich bekannt (DE-A 3 346 573; EP-A 153 083; EP-A 162 695) und können beispielsweise durch Umsetzung von 4-Amino-6-methoxy-1,3,4,5-tetrahydrobenz[c,d]indol und 2-(4-Brombutyl-1,2-benzisothiazol-3(2H)-on-1,1-dioxid hergestellt werden.

Aus der Gruppe der Indolamine sind insbesondere die Tryptamin-Derivate der Formel

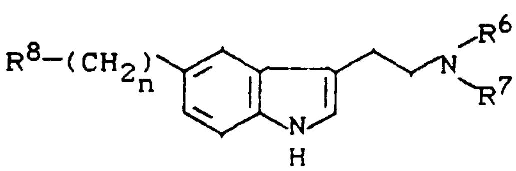

wobei

n für 1 und

$R^8$ für $CONR^1R^2$ oder $SO_2NR^1R^2$ steht, wobei $R^1$, $R^2$ die oben angegebene Bedeutung haben, und

$R^6$, $R^7$ gleich oder verschieden sind und Wasserstoff oder Niederalkyl ($C_1$ bis etwa $C_6$) bedeuten,

<u>oder</u>

n für die Zahl O,

$R^8$ für $CONR^1R^2$ steht, wobei $R^1$, $R^2$ die oben angegebene Bedeutung haben und

$R^6$, $R^7$ die oben angegebene Bedeutung haben,

oder

n für die Zahl O,

$R^8$ für H, OH, $OCH_3$ steht,

$R^6$ Wasserstoff oder Methyl bedeutet und

$R^7$ für den Rest

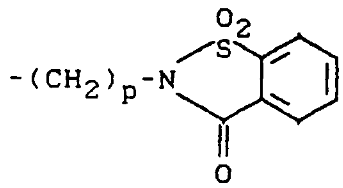

steht,

wobei p für die Werte 2, 3, 4 oder 5 steht,

und/oder deren Salze

bevorzugt.

Beispielsweise seien die folgenden Wirkstoffe genannt:

5-Carboxamidotryptamin, N,N-Dipropyl-5-carboxamidotryptamin, 3-(2-Aminoethyl)-1H-indol-5-(N-methyl)-acetamid (AH 25 086), 3-(2-Dimethylaminoethyl)-1H-indol-5-(N-methyl)methansulfonamid (GR 43 175), 3-(2-[4-[2-(1,2-Benzisothiazol-3(2H)-on-1,1-dioxido)]butyl]amino)ethyl-5-methoxy-1H-indol.

Insbesondere bevorzugt wird hiervon N,N-Dipropyl-5-carboxamidotryptamin und 3-(2-Dimethylaminoethyl)-1H-indol-5-(N-methyl)methansulfonamid.

Die Tryptamin-Derivate sind an sich bekannt (Fozard, Trends in Pharmacological Sciences 8, 501 (1987); Scrip's Serotonin-Report, PJB-Publications (1988); EP-A 236 930; DE-A 3 131 728; DE-A 2 940 687, DE-A 3 320 521) und können beispielsweise durch Umsetzung von 3-(2-Bromethyl)-1H-indol-5-(N-methyl)-methansulfonamid mit Dimethylamin hergestellt werden.

Aus der Gruppe der Aminoalkyl-benzodioxane sind insbesondere die 2-Aminomethyl-Derivate der Formel

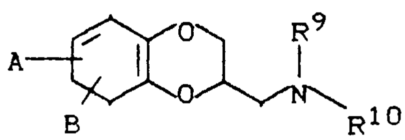

wobei A, B für H oder gemeinsam für -CH = CH-CH = CH- stehen und einen Ring bilden,

$R^9$ Wasserstoff oder Methyl bedeutet,

$R^{10}$

für 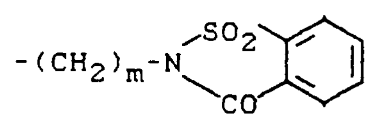 , 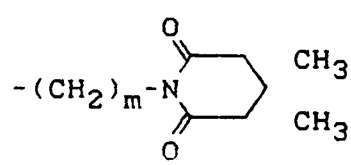 oder

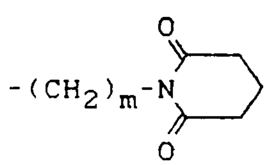

wobei m für die Zahlen 2, 3, 4 oder 5 steht

oder $-NR^9R^{10}$ für [1-Phenyl-1,3,8-triazaspiro[4,5]decan-4-on]-8-yl steht,

und/oder deren Salze

bevorzugt.

Beispielsweise seien die folgenden Wirkstoffe genannt:
8-(1,4-Benzodioxan-2-yl-methyl)-1-phenyl-1,3,8-triazaspiro[4,5]decan-4-on (nach INN: Spiroxatrin), 8-[4-(1,4-benzodioxan-2-ylmethylamino)butyl]-8-azaspiro[4,5]decan-7,9-dion (MDL 72832), 2-[4-(1,4-benzodioxan-2-ylmethylamino)butyl]-1,2-benzisothiazol-3(2H)-on-1,1-dioxid.

Insbesondere bevorzugt wird hiervon 2-[4-(1,4-benzodioxan-2-ylmethylamino)butyl]-1,2-benzisothiazol-3-(2H)-on-1,1-dioxid.

Die 2-Aminomethyl-benzodioxan-Derivate sind an sich bekannt (EP-A 236 930; Fozard, Trends in Pharmacological Sciences 8, 501 (1987); Scrip's Serotonin-Report, PJB-Publications (1988); EP 170 213) und können beispiels-weise durch Umsetzung von 2-Aminomethylbenzodioxan mit 2-(4-Brombutyl)-1,2-benzisothiazol-3(2H)-on-1,1-dioxid hergestellt werden.

Aus der Gruppe der Amino-tetraline sind besonders die 2-Amino-Derivate der Formel

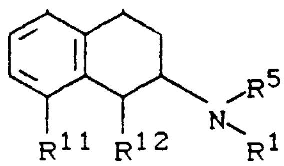

wobei $R^{12}$ für H, $CH_3$ steht,
$R^1$, $R^5$ die oben angegebene Bedeutung haben und
wenn $R^{12}$ = H ist,
und
falls $R^5$ = Wasserstoff, Alkyl ($C_1$ bis $C_6$) ist,
$R^{11}$ für OH, $OCH_3$, $NH_2$, $OCOR^1$, $NHCOR^1$ oder $NHSO_2CH_3$ steht, wobei $R^1$ die oben angegebene Bedeutung hat,
oder
falls $R^5$ die oben angegebene Bedeutung von -Y-Z be
sitzt,
$R^{11}$ für OH, $OCH_3$ steht,
oder
falls $R^5$ für Alkyl ($C_1$ bis $C_6$) steht,
$R^{11}$ für -Y-Z oder -O-Y-Z steht, wobei -Y-Z die oben angegebene Bedeutung hat,
oder
wenn $R^{11}$ = $CH_3$ ist,
$R^1$, $R^5$ für n-Propyl stehen
und/oder deren Salze bevorzugt.

Beispielsweise seien die folgenden Wirkstoffe genannt:
2-(N,N-Dipropylamino)-8-hydroxy-1,2,3,4-tetrahydronaphthalin, 2-{4-[2-(1,2-benzisothiazol-3(2H)-on-1-dioxido)]butyl}amino-8-methoxy-1,2,3,4-tetrahydronaphthalin.

Insbesondere bevorzugt wird hiervon 2-(N,N-Dipropylamino)-8-hydroxy-1,2,3,4-tetrahydronaphthalin.

Die Aminotetralin-Derivate sind an sich bekannt (EP-A 41 488; EP-A 236 930; Arvidsson et. al. J. Med. Chem. 30, 2105, 1987) und können beispielsweise durch Umsetzung von 8-Methoxy-2-tetralon mit Dipropylamin unter reduzierenden Bedingungen hergestellt werden.

Aus der Gruppe der Amino-chromane und -thiopyrane sind beispielsweise die 3-Aminochroman- und -thiopyran- Derivate der Formel

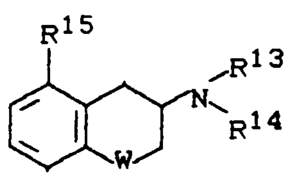

wobei W für Sauerstoff oder Schwefel steht,
$R^{13}$, $R^{14}$ die für $R^1$, $R^2$ angegebene Bedeutung haben,

für Aralkyl ($C_7$ bis $C_{18}$) stehen oder gemeinsam einen carbocyclischen Ring ($C_4$ bis $C_7$) bilden und
$R^{15}$ für H, OH oder O-Alkyl ($C_1$ bis $C_6$) steht
und/oder deren Salze
bevorzugt.

Beispielsweise seien die folgenden Wirkstoffe genannt:
3-N,N-Dipropylamino-5-hydroxy-thiochroman, 3-N,N-Dipropylamino-5-ethoxy-thiochroman, 3-N,N-Dipropylamino-5-ethoxy-chroman.

Insbesondere bevorzugt wird hiervon 3-N,N-Dipropylamino-5-hydroxy-thiochroman.

Die 3-Aminochroman- und -thiopyran-Derivate sind an sich bekannt (EP-A 222 996) und können beispielsweise durch Umsetzung von 3,4-Dihydro-5-methoxy-2H-benzothiopy-ran-3-amin mit Propyliodid hergestellt werden.

Aus der Gruppe der Indolylalkylpiperidine sind insbesondere die 1-[2-(3-Indolyl)]ethyl-Derivate der Formel

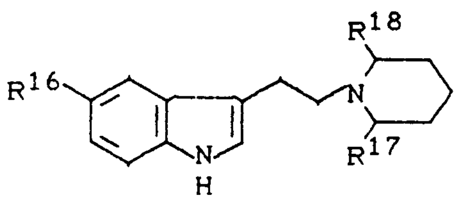

wobei $R^{16}$ für H, Halogen, Methyl, CN oder $CONH_2$ steht und
$R^{17}$, $R^{18}$ gleich oder verschieden sind und für Methyl oder Ethyl stehen,
und/oder deren Salze
bevorzugt.

Beispielsweise seien die folgenden Wirkstoffe genannt:
1-[2-(3-Indolyl)]-ethyl-2,6-dimethyl-piperidin, 1-{2-[3-(5-carboxamido)indolyl]}ethyl-2,6-dimethyl-piperidin.

Insbesondere bevorzugt wird hiervon
1-{2-[3-(5-carboxamido)indolyl)]}ethyl-2,6-dimethyl-piperidin.

Die 1-[2-(3-Indolyl)]ethyl-piperidin-Derivate sind an sich bekannt (DE-A 34 30 284) und können beispielsweise durch Umsetzung von 1-{2-[3-(5-Brom)indolyl]}-ethyl-2,6-dimethylpiperidin mit Natriumcyanid hergestellt werden.

Aus der Gruppe der Tetrahydropyridine sind beispielsweise die Indolyl-tetrahydropyridin-Derivate der Formel

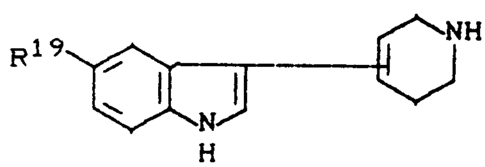

wobei $R^{19}$ für H, $OCH_3$, O-Ethyl, O-Propyl oder Halogen steht,
und/oder deren Salze
bevorzugt.

Beispielsweise seien die folgenden Wirkstoffe genannt:
5-Methoxy-3-(1,2,3,6-tetrahydropyridin-4-yl]-1H-indol (RU 24 924),
5-Methoxy-3-(1,2,3,6-tetrahydropyridin-5-yl)-1H-indol.

Insbesondere bevorzugt wird hiervon 5-Methoxy-3-(1,2,3,6-tetrahydropyridin-4-yl)-1H-indol.

Die Indolyl-tetrahydropyridin-Derivate sind an sich bekannt (EP-A 3 199; SCRIP's Serotonin Report, PJB-Publications (1988); Fozard, Trends in Pharmacological Sciences 8 501 (1987)) und können beispielsweise durch Umsetzung von 4-Piperidon mit 5-Methoxyindol hergestellt werden.

Gegenstand der vorliegenden Erfindung ist die Verwendung von einem oder mehreren Wirkstoffen mit Serotonin-agonistischer Wirkung, der bei der Bindung an $5HT_{1A}$-Rezeptoren eine Bindungsstärke von kleiner als 10.000 nmol/l aufweist zur Herstellung von Arzneimitteln zur Behandlung der Apoplexia cerebri. Die erfindungsgemäßen Arzneimittel enthalten im allgemeinen 0,01 bis 20 Gew.-%, bevorzugt 0,1 bis 10

Gew.-%, des oder der Wirkstoffe, bezogen auf die Zubereitung.

Die Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungs mittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispersionsmittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und stoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Die Wirkungsweise der erfindungsgemäßen Wirkstoffe zur präventiven und nachfolgenden Behandlung von Apoplexia cerebri läßt sich anhand der Methode der fokalen cerebralen Ischämie bei der Ratte als Schlaganfallmodell (Middle Cerebral Artery Occlusion (MCA)) nachweisen. Literatur: A. Tamura et. al.; J. Cerebral Blood Flow and Metab. 1, 53-60, 1981.

Die Behandlung von Apoplexia cerebri mit den erfindungsgemäßen Wirkstoffen stellt ein neues und überraschendes Behandlungsprinzip dar.

## Beispiele

### Testmodell

1. Verschluß der Arteria cerebri media bei der Ratte

In Narkose ($N_2O$/Halothan) wird in der Mitte zwischen Auge und Ohr ein Hautschnitt gelegt und unter stumpfer Durchtrennung der darunterliegenden Muskelpartien ein Zugang zum Schädel im Bereich des Foramen ovale geschaffen. Etwa 1 mm rostrodorsal dieser Öffnung wird der Schädelknochen entfernt, die darunterliegenden Hirnhäute eröffnet, und durch Elektrokoagulation die Arteria cerebri media verschlossen. Daraufhin wird die Muskulatur in ihre ursprüngliche Lage gebracht und die durchtrennte Hautpartie wieder vernäht. Die Tiere werden in ihren Heimatkäfig zurückgesetzt. Im Verlauf der folgenden Stunden entwickelt sich im Mediastromgebiet ein Infarkt, dessen Ausdehnung histologisch quantifiziert wird.

2. Quantifizierung der Infarktgröße

Die Gehirne werden 48 Stunden nach dem Gefäßverschluß durch Perfusion mit phosphatgepufferter (pH 7,4) Formalinlösung fixiert, aus dem Schädel genommen, in Paraplast eingebettet und in dünne Schnitte (8 µm) zerlegt. Die Gehirnschnitte werden zur Unterscheidung zwischen intaktem und geschädigtem Gewebe mit Kresylviolett gefärbt. Auf ca. 15 Schnitten im Abstand von 0,5 mm werden jeweils die Flächen für die gesamte Hirnhälfte, den gesamten Cortex, das gesamte Striatum und die entsprechenden geschädigten Gebiete mit Hilfe eines Bildanalysesystems planimetrisch bestimmt. Aus den Flächen aufeinanderfolgender Schnitte und den jeweiligen Abständen wird dann das Infarktvolumen bestimmt, und zwar getrennt nach Cortex, Striatum und Gesamtvolumen.

3. Arzneistoffbehandlung

Die Tiere wurden im allgemeinen 30 Minuten vor dem Gefäßverschluß mit Arzneistoff behandelt. Die Stoffe wurden in physiologischer Kochsalzlösung aufgenommen und in einem Volumen von 1 ml/kg intraperitoneal appliziert. Die Dosierung lag zwischen 1 und 30 mg/kg Körpergewicht. In einer weiteren Serie wurde Ipsapiron (30 mg/kg) 1 Stunde nach dem Gefäßverschluß intraperitoneal verabreicht. Ergebnisse der Untersuchungen sind beispielhaft in der Tabelle gezeigt. Das Infarktvolumen wird in Anwesenheit der Arzneistoffe jeweils signifikant reduziert.

Nach diesem Test wurden die folgenden Wirkstoffe untersucht:

| Beispiel | Wirkstoff | Infarktvolumen im Cortex ohne Behandlung | Infarktvolumen im Cortex mit Behandlung |
|---|---|---|---|
| 1 | 8-OH-DPAT (1 mg/kg) | 61.7+/-10.4 | 40.7+/-15.5* |
| 2 | Bay R 1531 (1 mg/kg) | 61.7+/-10.4 | 24.0+/-18.6*** |
| 3 | Buspiron (10 mg/kg) | 66.1+/-12.9 | 38.5+/-14.4** |
| 4 | Gepiron (10 mg/kg) | 77.2+/-32.3 | 39.6+/-31.4* |
| 5 | Ipsapiron (10 mg/kg) | 70.0+/-21.4 | 40.8+/-23.5* |
| 6 | Ipsapiron (30 mg/kg) | 36.5+/-12.2 | 13.7+/- 5.4*** |
| 7 | Ipsapiron (30 mg/kg; (1 Stunde nach MCA-Okklusion) | 66.1+/-12.9 | 31.5+/-20.9** |

* = p<0.05 ** = p<0.01 *** = p<0.001 (H-Test bzw. U-Test)

## Patentansprüche

1. Verwendung von Wirkstoffen, mit Serotonin-agonistischer Wirkung, die bei der Bindung an $5HT_{1A}$-Rezeptoren eine Bindungsstärke von kleiner als 10 000 nmol/l aufweisen, zur Herstellung von Arzneimitteln zur Behandlung von Apoplexia cerebri.

2. Verwendung nach Anspruch 1, dadurch gekennzeichnet, daß die Wirkstoffe eine Bindungsstärke von kleiner als 1000 nmol/l aufweisen.

3. Verwendung nach Anspruch 1, dadurch gekennzeichnet, daß die Wirkstoffe eine Bindungsstärke von 0,1 bis 100 nmol/l aufweisen.

4. Verwendung nach den Ansprüchen 1 bis 3, dadurch gekennzeichnet, daß Wirkstoffe aus der Gruppe der Aryl- und Hetaryl-piperazine eingesetzt werden.

5. Verwendung nach den Ansprüchen 1 bis 3, dadurch gekennzeichnet, daß Wirkstoffe aus der Gruppe der Aminotetrahydrobenzindole eingesetzt werden.

6. Verwendung nach den Ansprüchen 1 bis 3, dadurch gekennzeichnet, daß Wirkstoffe aus der Gruppe der Indolamine eingesetzt werden.

7. Verwendung nach den Ansprüchen 1 bis 3, dadurch gekennzeichnet, daß Wirkstoffe aus der Gruppe der Aminoalkyl-benzodioxane eingesetzt werden.

8. Verwendung nach den Ansprüchen 1 bis 3, dadurch gekennzeichnet, daß Wirkstoffe aus der Gruppe der Aminotetraline eingesetzt werden.

9. Verwendung nach den Ansprüchen 1 bis 3, dadurch gekennzeichnet, daß Wirkstoffe aus der Gruppe der Aminochromane und -thiopyrane eingesetzt werden.

10. Verwendung nach den Ansprüchen 1 bis 3, dadurch gekennzeichnet, daß Wirkstoffe aus der Gruppe der Indolyl-alkyl-piperidine eingesetzt werden.

11. Verwendung nach den Ansprüchen 1 bis 3, dadurch gekennzeichnet, daß Wirkstoffe aus der Gruppe der Tetrahydropyridine eingesetzt werden.

## Claims

1. Use of active compounds which act as serotonin agonists and have a binding strength of less than 10,000 nmol/l on binding to $5HT_{1A}$ receptors for the preparation of medicaments for the treatment of cerebral apoplexy.

2. Use according to Claim 1, characterized in that the active compounds have a binding strength of less than 1,000 nmol/l.

3. Use according to Claim 1, characterized in that the active compounds have a binding strength from 0.1 to 100 nmol/l.

4. Use according to Claims 1 to 3, characterized in that active compounds from the group of aryl- and hetarylpiperazines are used.

5. Use according to Claims 1 to 3, characterized in that active compounds from the group of aminotetrahydrobenzindoles are used.

6. Use according to Claims 1 to 3, characterized in that active compounds from the group of indolamines are used.

7. Use according to Claims 1 to 3, characterized in that active compounds from the group of aminoalkylbenzodioxanes are used.

8. Use according to Claims 1 to 3, characterized in that active compounds from the group of aminotetralins are used.

9. Use according to Claims 1 to 3, characterized in that active compounds from the group of aminochromans and -thiopyrans are used.

10. Use according to Claims 1 to 3, characterized in that active compounds from the group of indolylalkylpiperidines are used.

11. Use according to Claims 1 to 3, characterized in that active compounds from the group of tetrahydropyridines are used.

**Revendications**

1. Utilisation de substances actives exerçant une activité agoniste vis-à-vis de la sérotonine, qui présentent, lors de la liaison à des récepteurs $5HT_{1A}$, une force de liaison inférieure à 10.000 nmoles/l, pour la préparation de médicaments destinés au traitement de l'apoplexie cérébrale.

2. Utilisation selon la revendication 1, caractérisée en ce que les substances actives présentent une force de liaison inférieure à 1.000 nmoles/l.

3. Utilisation selon la revendication 1, caractérisée en ce que les substances actives présentent une force de liaison de 0,1 à 100 nmoles/l.

4. Utilisation selon la revendication 1 à 3, caractérisée en ce qu'on met en oeuvre des substances actives choisies parmi le groupe des aryl- et hétaryl-pipérazines.

5. Utilisation selon la revendication 1 à 3, caractérisée en ce qu'on met en oeuvre des substances actives choisies parmi le groupe des aminotétrahydrobenzindoles.

6. Utilisation selon la revendication 1 à 3, caractérisée en ce qu'on met en oeuvre des substances actives choisies parmi le groupe des indolamines.

7. Utilisation selon la revendication 1 à 3, caractérisée en ce qu'on met en oeuvre des substances actives choisies parmi le groupe des aminoalkylbenzodioxannes.

8. Utilisation selon la revendication 1 à 3, caractérisée en ce qu'on met en oeuvre des substances actives choisies parmi le groupe des aminotétralines.

9. Utilisation selon la revendication 1 à 3, caractérisée en ce qu'on met en oeuvre des substances actives choisies parmi le groupe des amino-chromanes et -thiopyrannes.

10. Utilisation selon la revendication 1 à 3, caractérisée en ce qu'on met en oeuvre des substances actives choisies parmi le groupe des indolyl-alkyl-pipéridines.

11. Utilisation selon la revendication 1 à 3, caractérisée en ce qu'on met en oeuvre des substances actives choisies parmi le groupe des tétrahydropyridines.